# EUROPEAN PATENT SPECIFICATION

(11) **EP 0 496 729 B1**
(45) Date of publication and mention of the grant of the patent: **22.06.1994**
(21) Application number: 89912649.4
(22) Date of filing: 26.10.1989
(51) Int. Cl.: A61K 31/12

(54) **MEDICAMENT FOR THE TREATMENT OF CRYPTOSPORIDIOSIS**
ARZNEIMITTEL ZUR BEHANDLUNG VON KRYPTOSPORIDIOSE
MEDICAMENT POUR LE TRAITEMENT DE LA CRYPTOSPORIDIOSE

(30) Priority: 16.10.1989 GB 8923254
(43) Date of publication of application: 05.08.1992
(73) Proprietor: THE WELLCOME FOUNDATION LIMITED, London NW1 2BP (GB)
(72) Inventor: LATTER, Victoria, Susan, Beckenham, Kent BR3 3BS (GB)
(74) Representative: Stott, Michael John
(86) International application number: GB8901287
(87) International publication number: WO9105550

(56) References cited:
- EP-A- 0 123 238
- EP-A- 0 123 239
- Int. Congr. Ser. - Excerpta Med., vol. 750, (Pharmacology), 1987, Elsevier Science Publishers B.V. (Biomedical Division), V.S. Latter pages 661-664
- T.M. Jones: "Presentation at interims", 27 April 1989, see second page

## Description

The present invention relates to the manufacture of medicaments for use in treatment and prophylaxis of cryptosporidiosis. More particularly the invention is concerned with the use of 2-[4-(4-chlorophenyl)cyclohexyl]-3-hydroxy-1,4-naphthoquinone and physiologically acceptable salts and other physiologically functional derivatives thereof in the manufacture of medicaments for use in treatment and prophylaxis of cryptosporidiosis.

Cryptosporidiosis is an infection caused by organisms of the genus Cryptosporidium e.g. C.parvum. These parasitic organisms have been found in a variety of mammalian species, including cows, sheep, horses, pigs and humans. The parasite is usually found in the intestine, where it attaches to the microvilli of the villus epithelial cells, leading to villus atrophy causing impaired digestion and adsorption, and diarrhoea. In animal husbandry, cryptosporidiosis is found to occur particularly in new-born animals, especially calves. Presently available treatments, which include sulphadimidine and sulphaquinoxaline, have been found unreliable.

Cryptosporidia have also been found in reservoirs and it appears that they are not eradicated by current water purification methods. Contamination of the drinking water supply has been known to lead to widespread outbreaks of cryptosporidiosis in the human population.
In humans, infection with cryptosporidia can cause fever, nausea, abdominal cramps, vomiting and severe diarrhoea, with the additional risk of dehydration and hypotension.
Patients in an already weakened state, such as children and the elderly, are thus particularly vulnerable to infections caused by cryptosporidia. Cryptosporidiosis can also be an extremely debilitating and complicating factor in immunocompromised patients (i.e. those with a defective or deficient immune system), who may be suffering from a number of different infections. There is a variety of circumstances in which the immune system may be defective or deficient. Thus, for example, immune system deficiency is common in immature or premature infants (neonates). It may also result from suppression by certain drugs, which may be deliberate e.g. in certain patients receiving organ transplants, or unavoidable e.g. as a side-effect of cancer chemotherapy. Disordered growth of one or more constituent parts of the immune system, e.g. as in certain forms of cancer, may also result in immunodeficiency. Immune deficiency may furthermore be caused by viral infections, including human immunodeficiency virus (HIV). Cryptosporidiosis is recognised as an HIV-associated opportunistic infection.

Although one study has reported treatment of cryptosporidiosis in AIDS patients with erythromycin and spiromycin there is presently no definitive drug therapy available for this infection and treatment is generally concerned with alleviating the symptoms rather than combating the causative organism. There is thus a need for chemotherapeutic agents to combat cryptosporidia.

The compound 2-[4-(4-chlorophenyl]cyclohexyl]3-hydroxy-1,4-naphthoquinone has previously been disclosed, for example in European Patent No. 123,238 which relates to 2-substituted-3-hydroxy-1,4-naphthoquinones of formula (I) 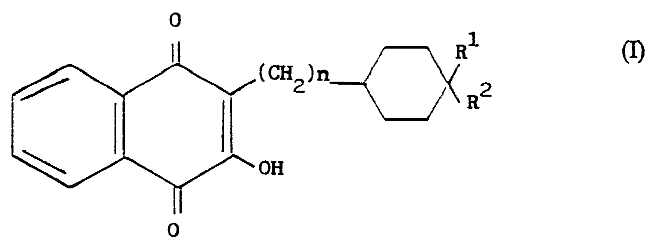
wherein either R¹ is hydrogen and R² is selected from C₁₋₆ alkoxy, aralkoxy, C₁₋₆ alkyl-C₁₋₆ alkoxy, phenyl substituted by one or two groups selected from halogen and C₁₋₆ alkyl, halogen and perhalo-C₁₋₆ alkyl or R¹ and R² are both C₁₋₆ alkyl or phenyl, and n is zero or 1, and physiologically acceptable salts thereof The compounds are said to have antiprotozoal activity. Specifically, compounds of formula (I) wherein n is zero are said to be active against the human malaria parasite Plasmodium falciparum and also against Eimeria species such as E.tenella and T.parva. Amongst the compounds specifically named and exemplified is the compound of formula (I) wherein n is zero, R¹ is hydrogen and R² is 4-chlorophenyl, i.e. 2-[4-(4-chlorophenyl)cyclohexyl]-3-hydroxy-1,4-naphthoquinone.

T.M. Jones "Presentation at Interims" 27 April 1989 stated "Wellcome has for many years, enjoyed an enviable reputation for its understanding of parasitic diseases and I am pleased to report that our anti-malarial compound has now passed the first test in man having shown good blood levels. We are now moving this compound into full clinical evaluation" and "Interestingly, several human infections are related to protozoal parasitic diseases (e.g. PCP, Toxoplasmosis, and diseases due to Cryptosporidium). We have preliminary, laboratory evidence that compounds such as our antimalarial inhibit these micro-organisms and we have an active programme of work to examine their potential in this regard".

A paper by V.S. Latter in Int.Congr.Ser.- Excerpta Med., vol. 750, 1987, p.661-664 describes Wellcome's development of 2-(4-tert-butyl cyclohexyl)-3-hydroxy-1,4-naphthoquinone as an antimalarial to the human volunteer study stage the metabolic problems that halted its development, and the discovery that the compound 2-[4-(4-chlorophenyl)cyclohexyl]-3-hydroxy-1,4-naphthoquinone appeared to be free of those metabolic problems and active against Plasmodium falciparum.

It has now surprisingly been found that 2-[4-(4-chlorophenyl) cyclohexyl]-3-hydroxy-1,4-naphthoquinone, represented in this specification by formula (II): 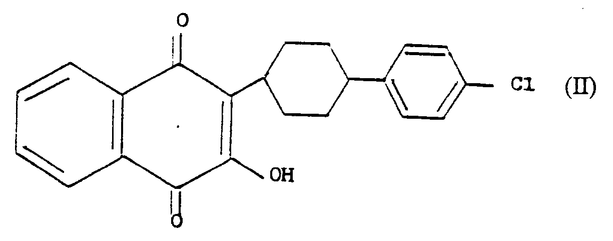
exhibits activity in vivo against experimental cryptosporidial infections in rats. In a first aspect the present invention provides the use of the compound of formula (II) and physiologically acceptable salts and other physiologically function derivatives thereof for the manufacture of a medicament for the treatment and/or prophylaxis of cryptosporidiosis in mammals (including humans).

Prevention of cryptosporidiosis is particularly important in an immunocompromised host, as discussed hereinabove. In the case of immunosuppression resulting from HIV infection, prophylaxis may be required by those diagnosed as seropositive for HIV i.e. having antibodies to HIV, and those with PGL (progressive generalised hymphadenopathy) or ARC (AIDS-related complex) as well as patients suffering from AIDS.

The hydroxyl group in the compound of formula (II) may form salts with appropriate bases, and physiologically acceptable salts of the compound (II) include inorganic base salts such as alkali metal (e.g. sodium and potassium) salts and alkaline earth metal (e.g. calcium) salts; organic bases salts e.g. phenylethylbenzylamine, dibenzylethyl-enediamine, ethanolamine and diethanolamine salts; and amino acid salts e.g. lysine and arginine.

Physiologically functional derivatives are derivatives which are converted in vivo, either by the host or the parasite to a compound of formula (II).

It will be appreciated that the compound of formula (II) may exist as the cis or trans isomer, that is to say that the cyclohexyl ring may be cis or trans substituted by the naphthoquinone nucleus and the chlorophenyl group. Both cis and trans isomers and mixtures thereof in any ratio may be used in accordance with the present invention. In general when the compound is in the form of a mixture of isomers the trans isomer will be present in an amount of about 50% or will be the predominant isomer but the use of mixtures in which the cis isomer predominates is also included within the scope of the invention. The specific ratio of isomers may be varied as required; typical mixtures include those in which the cis/trans isomer ratio is about 1:1,40:60 and 5:95. For use according to the present invention the trans isomer of the compound of formula (II) or a mixture of its cis and trans isomers containing at least 95% e.g. 99% of the trans isomer is preferred.

The compound of formula (II) may also exist in a tautomeric form in which the hydroxyl group donates its proton to one of the oxo groups and the use of such tautomeric forms is included within the scope of this invention. However, it is believed that the stable form is that shown in formula (II).

It will be appreciated that the amount of the compound of formula (II) or its salt or other physiologically functional derivatives required in a medicament for use in the treatment or prophylaxis of cryptosporidiosis will depend inter alia on the route of administration, the age and weight of the mammal (e.g. human) to be treated and the severity of the condition being treated. In general, a suitable dose for administration to man in a medicament for use in treatment of cryptosporidiosis is the range of 1.0mg to 200 mg per kilogram bodyweight per day, for example from 10mg/kg to 100mg/kg₇ particularly 25 to 100 mg/kg. It will be appreciated that for administration to neonates₇ lower doses may be required.

For use in prophylactic treatment a medicament comprising the compound of formula (II) or its salt or other physiologically functional derivative may also be given less frequently, e.g. as a single dose on alternate days, once or twice per week or once or twice per month. The dosage in a medicament for use in prophylactic treatment will depend inter alia on the frequency of administration, and, where a depot preparation or controlled release formulation is used the rate of release of the active ingredient. Thus for once-weekly administration a suitable prophylactic dose of the component is in the range of 0.1 to 100 mg/kg, e.g. 0.5 to 50 mg/kg particularly 5 to 50 mg/kg.

It should be understood that the dosages referred to above are calculated in terms of the compound of formula (II) per se.

For use according to the present invention the compound of formula (II) or a physiologically acceptable salt or other physiologically functional derivative thereof is preferably presented as a pharmaceutical formulation.

Pharmaceutical formulations comprise the active ingredient (that is, the compound of formula (II) or a physiologically acceptable salt or other physiologically functional derivative thereof) together with one or more pharmaceutically acceptable carriers therefor and optionally other therapeutic and/or prophylactic ingredients. The carrier(s) must be acceptable in the sense of being compatible with the other ingredients of the formula and not deleterious to the recipient thereof.

The compound of formula (II) or its salt or other physiologically functional derivative may conveniently be presented as a pharmaceutical formulation in unit dosage form. A convenient unit dose formulation contains the active ingredient in an amount of from 10 mg to 3g, e.g. 50mg to 3g. A typical unit dose may contain for example 50mg, 1g, 2, or 3g of the active ingredient.

Pharmaceutical formulations include those suitable for oral, topical (including dermal, buccal and sublingual), rectal and parenteral (including subcutaneous, intradermal, intramuscular and intravenous) administration, as well as administration by naso-gastric tube. The formulation may, where appropriate, be conveniently presented in discrete dosage units and may be prepared by any of the methods well known in the art of pharmacy. All methods include the step of bringing into association the compound of formula (II) or a physiologically acceptable salt or other physiologically functional derivative thereof with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of the active ingredient. A tablet may be made by compression or moulding, optionally with one or more accessory ingredients. Compressed tablets may be prepared by compressing in a suitable machine the active ingredient in a free-flowing form such as a powder or granules optionally mixed with a binder, lubricant, inert diluent, lubricating agent, surface-active agent or dispersing agent. Moulded tablets may be made by moulding an inert liquid diluent. Tablets may be optionally coated and, if uncoated, may optionally be scored. Capsules may be prepared by filling the active ingredient, either alone or in admixture with one or more accessory ingredients, into the capsule shells and then sealing them in the usual manner. Cachets are analogous to capsules wherein the active ingredient together with any accessory ingredient(s) is sealed in a rice paper envelope. The compound of formula (II) or a physiologically acceptable salt or other physiologically functional derivative thereof may also be formulated as dispersible granules, which may for example be suspended in water before administration, or sprinkled on food. The granules may be packaged e.g. in a sachet. Formulations suitable for oral administration wherein the carrier is a liquid may be presented as a solution or a suspension in an aqueous liquid or a non-aqueous liquid, or as an oil-in-water liquid emulsion.

Formulations for oral administration include controlled release dosage forms e.g. tablets wherein the active ingredient is formulated in an appropriate release - controlling matrix, or is coated with a suitable release - controlling film. Such formulations may be particularly convenient for prophylactic use.

The active ingredient may also be formulated as a solution or suspension suitable for administration via a naso-gastric tube.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the active ingredient with the softened or melted carrier(s) followed by chilling and shaping in moulds.

Pharmaceutical formulations suitable for parenteral administration include sterile solutions or suspensions of the active ingredient in aqueous or oleaginous vehicles. Injectable preparations may be adapted for bolus injection or continuous infusion. Such preparations are conveniently presented in unit dose or multi-dose containers which are sealed after introduction of the formulation until required for use. Alternatively, the active ingredient may be in powder form which is constituted with a suitable vehicle, such as sterile, pyrogen-free water, before use.

The compound of formula (II) or a physiologically acceptable salt or other physiologically functional derivative thereof may also be formulated as a long-acting depot preparation, which may be administered by intramuscular injection or by implantation e.g. subcutaneously or intramuscularly.Depot preparations may include, for example, suitable polymeric or hydrophobic materials, or ion-exchange resins. Such long-acting formulations are particularly convenient for prophylactic use.

It should be understood that in addition to the aforementioned carrier ingredients the pharmaceutical formulations for the various routes of administration described above may include, as appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

The compound of formula (II) or a salt or other physiologically functional derivative thereof may also be used in accordance with the present invention in combination or concurrently with other therapeutic agents, for example agents used in the treatment of immunocompromised patients, including antibacterial agents antifungal agents; anticancer agents such as interferons e.g. alpha-interferon; antiviral agents such as azidothymidine (AZT.zidovudine), immunostimulants and immunodulators. The compound of formula (II) may also be administered in combination with a 4-pyridinol compound, as described in EPA 123,239 e.g. 3,5-dichloro-2,6-dimethylpyridinol (meticlorpindol). The compound of formula (II) may also be administered in combination or concurrently with antidiarrhoeal agents such as loperamide hydrochloride and/or diphenoxylate hydrochloride, or with morphine sulphate. Oral rehydration therapy may also be carried out concurrently.

Compositions suitable for veterinary use include those adapted for oral, parenteral, and intrarumenal administration.

Compounds suitable for oral administration include drenches (oral liquid dosing), which may be solutions or suspensions; tablets, boluses, pastes, or in-feed preparations in the form of powders, granules or pellets.

Alternatively, veterinary compositions may be adapted to be administered parenterally by sub-cutaneous, intramuscular or intravenous injection of a sterile solution or suspension, by implantation or as an intramammary injection whereby a suspension or solution is introduced into the udder via the teat.

For intrarumenal injection, the compositions of the invention may be solutions or solid or microcapusule suspensions. Typically the compositions are similar to the oral liquid preparations or parenteral preparations described herein. Such compositions are injected directly into the rumen, usually through the side of the animal, for example by a hypodermic syringe and needle or by an automatic injection device capable of giving single or multiple doses.

For veterinary administration the compound of formula (II) or its salt or other physiologically functional derivative is preferably formulated with one or more veterinarily acceptable carriers.

For oral administration, fine powders or granules may contain diluting agents, for example lactose, calcium carbonate, calcium phosphate, mineral carriers, etc., dispersing and/or surface active agents, for example polysorbates such as Tweens or Spans (Tween and Span are registered Trade Marks), and may be presented in a drench, in water or in syrup, in a bolus, paste, or in a feed preparation, in capsules or sachets in the dry state or in a non-aqueous suspension or in a suspension in water or syrup. Where desirable or necessary, preserving, suspending, thickening or emulsifying agents can be included. If intended for oral use, a bolus will be provided with retention means to inhibit regurgitation, for example it may be weighted with a heavy density material such as iron or tungsten or the like or may be retained by its shape, for example by wings which spring after adminstration. Boluses may contain disintegrating agents such as maize starch or calcium or sodium methyl celluloses, hydroxypropylmethycellulose, guar based vegetable gums, sodium alginates or sodium starch glycolates; granulating or binding agents such as starch in the form of mucilage, starch derivatives, such as "Snow Flake" (Snow Flake is a registered Trade Mark), cellulose derivatives such as talc, calcium stearate, methyl cellulose, gelatin or polyvinylpyrrolidone: and/or lubricating agents, such as magnesium stearate or stearic acid.

For parenteral administration, the compounds may be presented in sterile injection solutions which may contain antioxidants or buffers, or as injectable suspensions. Suitable solvents include water, in the case of suspensions, and organic solvents such as dimethylformamide, dimethylacetamide, diethylacetamide, ethyl lactate, ethyl akate, dimethylsulphoxide, alcohols, e.g. ethanol, glycols, e.g. ethylene glycol, propylene glycol, butylene glycol and hexamethylene glycol, polyethylene glycols containing 2 to 159 ethylene glycol monomer units and having average molecular weights from about 90 to 7500, glycerin formal, glycofural, glycerol, isopropylmyristate N-methylpyrrolidone, 2-pyrrolidone polyethyiene glycoethers of tetrahydrofurfuryl alcohol and diethylene glycol, and fixed and neutral oils, for example fractionated coconut oil. Parenteral formulations may also contain isotonic agents.

For veterinary use the compound of formula (II) may be employed together with other therapeutic agents used in the field of animal health, for example with anticoccidial and/or antitheilerial agents.

Methods for preparing the compound of formula (II) are described in EP 123,238, and one specific method is illustrated in Example 3.

### Example 1

The following examples illustrate, with reference to the compound of formula (II) per se, pharmaceutical and veterinary formulations which may be employed in accordance with the present invention:
A. Injectable solution
A solution for intramuscular injection may be prepared by mixing:

| | |
|---|---|
| Compound of formula (II) | 9.5 parts by weight |
| Dimethyl sulphoxide | 19.0 parts by weight |
| Sorbitan monooleate | 4.5 parts by weight |
| Corn oil | 67.0 parts by weight |
| | 1̅0̅0̅.̅0̅ |

B. Injectable solution

| | |
|---|---|
| Compound of formula (II) | 5 parts by weight |
| N-methyl-pyrollidone | 48.3 parts by weight |
| Tween 80 (Tween is a registered Trade Mark) | 2 parts by weight |
| Span 80 (Span is a registered Trade Mark) | 4.7 parts by weight |
| Miglyol 812 (Miglyol is a registered Trade Mark) | 40 parts by weight |
| | 1̅0̅0̅.̅0̅ |

C. Tablet

| | |
|---|---|
| Compound of formula (II) | 25.0 mg |
| Lactose BP | 48.5 mg |
| Microcrystalline Cellulose BP ("Avicel pH 101") (Avicel is a registered Trade Mark) | 10.0 mg |
| Low-substituted Hydroxypropyl; Cellulose BP ("LHPC LH-11") (LHPC LH-11 is a registered Trade Mark) | 10.0 mg |
| Sodium Starch Glycollate BP ("Explotab") (Explotab is a registered Trade Mark) | 3.0 mg |
| Povidone BP ("K30") (K30 is a registered Trade Mark) | 3.0 mg |
| Magnesium Stearate BP | 0.5 mg |
| | 100.0 mg |

D. Oral Suspension

| | |
|---|---|
| Compound of formula (II) | 50 mg |
| Avicel RC 591 (Avicel is a registred Trade Mark) | 75 mg |
| Sucrose syrup | 3.5 mg |
| Methylhydroxybenzoate | 5 mg |
| Colour | 0.01% w/v |
| Cherry flavour | 0.1 % w/v |
| Tween 80 (Tween is a registered Trade Mark) | 0.2 % w/v |
| Water | to 5 ml |

E. Injectable suspension

| | |
|---|---|
| Compound of formula (II) | 100 mg |
| Polyvinyl pyrrolidone (PVP) | 170 mg |
| Tween 80 (Tween is a registered Trade Mark) | 0.2% v/v |
| Methylhydroxybenzoate | 0.1% w/v |
| Water for Injection | to 3 ml |

F. Capsule

| | |
|---|---|
| Compound of formula (II) | 100 mg |
| Starch 1500 | 150 mg |
| Magnesium stearate | 2.5 mg |
| filled into a hard gelatin capsule | |

G. Aqueous Suspension
An aqueous suspension may be prepared as follows:

| | |
|---|---|
| Compound of formula (II) | 1.00 part by weight |
| Neosyl (Neosyl is a registered Trade Mark) | 16.00 parts by weight |
| Bentonite | 3.20 parts by weight |
| Glycerin | 15.00 parts by weight |
| Sodium benzoate | 1.00 part by weight |
| Bevaloid 35/2 (Bevaloid is a registered Trade Mark) | 1.00 part by weight |
| Thymol 35/2 | 0.04 parts by weight |
| Water | 62.76 parts by weight |
| | 100.00 |

H. Salt Block
A salt block may be prepared by mixing a finely divided compound of formula (II) (0.5 parts by weight) with sodium chloride (99.5 parts by weight) and the mixture pressed into blocks.
I. Paste
The following paste may be prepared:

| | |
|---|---|
| Compound of formula (II) | 3.0 parts by weight |
| Gum tragacanth | 4.0 parts by weight |
| Bevaloid 35/3 (Bevaloid is a registered Trade Mark) | 1.0 part by weight |
| Nipagin "M" (Nipagin is a registered Trade Mark) | 0.1 parts by weight |
| Glycerin | 19.0 parts by weight |
| Water | 72.9 parts by weight |
| | 100.00 |

The use of the compound of formula (II) according to the present invention is illustrated by the following example:

### BIOLOGICAL TEST RESULTS

### Example 2

### Activity against Cryptosporidiosis

### Test Compound

### A: 2-[trans-4-chlorophenyl)cyclohexyl]-3-hydroxy-1,4-naphthoquinone.

### Method

Dexamethasone was administered to groups of rats (Bodyweight ca.200g) in the drinking water for 10 days prior to C.parvum oocyst inoculation. The animals received the test compound for 11 days from the time of oocyst inoculation. The test compound was administered orally by gavage at a dosage of 100mg/kg/day, and in the feed, which the animals consumed at a rate equivalent to ca. 115mg/kg of drug/day/ A control group of dexamethasone-treated rats received no test compound. At the end of the treatment period the number of cryptosporidia/ileal villus and oocyst shed in the faeces of the rats teated with the test compound were compared with the control group.

The results which are presented In Table 1 below indicate that the test compound, administered either by gavage or in the feed markedly reduced the level of infection as compared with controls.

**TABLE 1**

| | Test Compound | | Control |
|---|---|---|---|
| | Gavage | Feed | |
| No. of animals per group | 20 | 20 | 18 |
| No. of survivors per group | 20 | 17 | 16 |
| Organisms/villus (range) | 0-22 | 0-32 | 0-35 |
| (Organisms/villus (Mean) | 6.6 | 9.9 | 16.9 |
| Organisms/villus (Median) | 3 | 4 | 17.5 |

### Example 3

### 2-[trans-4-(4-Chlorophenyl)cyclohexyl]-3-hydroxy-1,4-naphthoquinone

a) 4-(4-Chlorophenyl)cyclohexane-1-carboxylic Acid
   Acetyl chloride (30g) and finely powdered aluminium chloride (60g) were stirred together in carbon disulphide (120 ml) and then cooled to -50^{o}C, in a CO₂/oxitol bath. Cyclohexene (30 g), previously cooled to -50^{o}C, was added dropwise during 10 minutes while maintaining the temperature of the reaction mixture at below -20^{o}C. The mixture was stirred at -50^{o}C for a further 60 minutes and the solvent then decanted to leave a gummy orange complex. A little chlorobenzene was added as the material warmed to ambient temperature; the remainder of the chlorobenzene (total 300 ml) was then added, the so-obtained solution heated at 40^{o}C for 3 hours with stirring, poured onto a mixture of ice and concentrated hydrochloric acid and the organic layer separated, washed with 2M hydrochloric acid, 2M sodium hydroxide and water, dried over anhydrous sodium sulphate and evaporated to dryness. The product was distilled in vacuo, the fraction boiling at 140-154^{o}C (0.1 mm Hg) collected, diluted with an equal volume of petroleum ether (40-60), cooled to -6^{o}C and a continuous stream of nitrogen gas bubbled through, and the separated colourless solid recovered.
   Bromine (2.8ml) was added to a solution of sodium hydroxide (6.2g) in water (42 ml) at 0^{o}C. The above-obtained substituted hexahydroacetophenone (3.1g) was dissolved in dioxan (15 ml) and the cold hypobromite solution then added, keeping the reaction mixture at below 20^{o}C. The reaction mixture was stirred at ambient temperature for 6 hours then allowed to stand overnight. Sodium metabisulphite was added to destroy excess hypobromite, the mixture cooled and then acidified to give a colourless solid. The solid was filtered off, washed with water, dried and recrystallised from ethanol to give 4-(4-chlorophenyl)cyclohexane-1-carboxylic acid, m.p. 254-256^{o}C.
b) 2-[4-(4-chlorophenyl)cyclohexyl]-3-chloro-1,4-naphthoquinone
   A mixture of 2-chloro-1,4-naphthoquinone (3.95g, 0.02 mol), 4-(4-chlorophenyl)cyclohexane-1-carboxylic acid (4.9g, 0.02 mol) and powdered silver nitrate (1.05g, 0.0062 mol) was heated to reflux with vigorous stirring in 40 ml of acetonitrile. A solution of ammonium persulphate (12.0g, 0.0525 mol) in 50 ml of water was added dropwise over 1 hour. The mixture was refluxed for 3 hours then cooled in ice for 30 mins, after which it was filtered, and the residual sticky solid extracted twice with boiling chloroform to remove inorganic material. The chloroform was removed by evaporation to leave a yellow-brown solid (ca 2.7g). This was dissolved in 40 ml of boiling acetonitrile; a little insoluble material was removed by filtration. On cooling, the title compound separated as yellow crystals, (550 mg) m.p. 172-175^{o}C.
   NMR, δH(d₆-DMSO) 8.05 (2H, mult., β-naphth), 7.85(2H, mult., α-naphth), 7.30 (4H, s., PhH), 3.30 (1H, br.t., CH), 2.67 (1H, br.t., CH), 1.2-2.4 (8H, mult., 4xCH₂).
c) 2-[4-(4-chlorophenyl)cyclohexyl]-3-hydroxy-1,4-naphthoquinone
   The product of stage (b) was suspended in 10 ml of boiling methanol and 0.55g of potassium hydroxide in 5.5 ml of water was added dropwise over 15 mins. The mixture was refluxed until a dark red solution formed, (after ca. 6 hrs) when 2 ml of concentrated hydrochloric acid was cautiously added dropwise. The mixture was cooled and filtered, and the solid residue washed thoroughly with water. The water washings were re-acidified and filtered. The combined solid residues (500 mg) mp 200-209^{o}, were recrystallised from acetonitrile to give the title product as the trans-isomer (300 mg) m.p. 216-219^{o}C.

## Claims

1. The use of 2-[4-(4-chlorophenyl)cyclohexyl]-3-hydroxy-1,4-naphthoquinone or a physiologically acceptable salt or other physiologically functional derivative thereof for the manufacture of a medicament for the treatment and/or prophylaxis of cryptosporidiosis in animals.

2. Use according to claim 1 wherein 2-[4-(4-chlorophenyl)cyclohexyl]-3-hydroxy-1,4-naphthoquinone is used in the form of its trans isomer or a mixture of its cis and trans isomers containing at least 95% of the trans isomer.

3. Use according to claim 1 or claim 2 wherein the medicament is adapted for the administration of 25 to 100 mg per kilogram of animal bodyweight per day of the compound or a physiologically acceptable salt or other physiologically functional derivative thereof for the treatment of cryptosporidiosis.

4. Use according to claim 1 or claim 2 wherein the medicament is adapted for the administration of 5 to 50 mg per kilogram of animal bodyweight per week of the compound or a physiologically acceptable salt or other physiologically functional derivative thereof for the prophylaxis of cryptosporidiosis.

5. Use according to any of claims 1 to 4 wherein the animal is a human.

6. Use according to claim 5 wherein the human is immunocompromised.

7. Use according to claim 6 wherein the human has an HIV infection.

## Patentansprüche

1. Verwendung von 2-[4-(4-Chlorphenyl)-cyclohexyl]-3-hydroxy-1,4-naphthochinon oder eines physiologisch annehmbaren Salzes oder eines anderen physiologisch funktionellen Derivats davon zur Herstellung eines Arzneimittels zur Behandlung und/oder Prophylaxe von Kryptosporidiose bei Tieren.

2. Verwendung nach Anspruch 1, worin 2-[4-(4-Chlorphenyl)-cyclohexyl]-3-hydroxy-1,4-naphthochinon in Form seines trans-Isomeren oder einer Mischung aus seinen cis- und trans-Isomeren, die mindestens 95 % des trans-Isomeren enthält, verwendet wird.

3. Verwendung nach Anspruch 1 oder Anspruch 2, worin das Arzneimittel zur Verabreichung von 25 bis 100 mg der Verbindung oder eines physiologisch annehmbaren Salzes oder eines anderen physiologisch funktionellen Derivats davon pro Kilogramm Körpergewicht des Tiers pro Tag bei der Behandlung von Kryptosporidiose angepaßt ist.

4. Verwendung nach Anspruch 1 oder Anspruch 2, worin das Arzneimittel zur Verabreichung von 5 bis 50 mg der Verbindung oder eines physiologisch annehmbaren Salzes oder eines anderen physiologisch funktionellen Derivats davon pro Kilogramm des Körpergewichts des Tiers pro Woche bei der Prophylaxe von Kryptosporidiose angepaßt ist.

5. Verwendung nach einem der Ansprüche 1 bis 3, worin das Tier ein Mensch ist.

6. Verwendung nach Anspruch 5, worin der Mensch ein beeinträchtigtes Immunsystem besitzt.

7. Verwendung nach Anspruch 6, worin der Mensch eine HIV-Infektion aufweist.

## Revendications

1. Utilisation de 2-[4-(4-chlorophényl)-cyclohexyl]-3-hydroxy-1,4-naphtoquinone ou d'un sel physiologiquement acceptable ou autre dérivé physiologiquement fonctionnel de celle-ci pour la fabrication d'un médicament pour le traitement et/ou la prophylaxie de la cryptosporidiose chez les animaux.

2. Utilisation suivant la revendication 1, dans laquelle la 2-[4-(4-chlorophényl)cyclohexyl]-3-hydroxy-1,4-naphtoquinone est utilisée sous la forme de son isomère trans ou d'un mélange de ses isomères cis et trans contenant au moins 95% de l'isomère trans.

3. Utilisation suivant la revendication 1 ou la revendication 2, dans laquelle le médicament est adapté pour l'administration de 25 à 100 mg par kg de poids de corps d'animal par jour du composé ou d'un sel physiologiquement acceptable ou autre dérivé physiologiquement fonctionnel de celui-ci pour le traitement de la cryptosporidiose.

4. Utilisation suivant la revendication 1 ou la revendication 2, dans laquelle le médicament est adapté pour l'administration de 5 à 50 mg par kg de poids de corps d'animal par semaine du composé ou d'un sel physiologiquement acceptable ou autre dérivé physiologiquement fonctionnel de celui-ci pour la prophylaxie de la cryptosporidiose.

5. Utilisation suivant l'une quelconque des revendications 1 à 4, dans laquelle l'animal est un humain.

6. Utilisation suivant la revendication 5, dans laquelle l'humain est immunocompromis.

7. Utilisation suivant la revendication 6, dans laquelle l'humain a une infection HIV.
